# EUROPEAN PATENT APPLICATION

(11) **EP 4 809 963 A1**
(43) Date of publication of application: **23.09.2026**
(21) Application number: 25165181.6
(22) Date of filing: 20.03.2025
(51) Int. Cl.: A61B 90/70, A61B 1/12

(54) **DEVICE FOR DISINFECTING AND WASHING FLEXIBLE AND RIGID ENDOSCOPES**

(71) Applicant: DUDEK Innovations Sp. z o.o., 80-426 Gdansk (PL)
(72) Inventor: DUDEK, Marek, 80-426 Gda sk (PL)
(74) Representative: Matyka, Malgorzata

(57) **Abstract**

The invention relates to a device for disinfecting and washing flexible and rigid endoscopes.

## Description

The invention relates to a device for disinfecting and washing flexible and rigid endosocpes.

Until now endoscopes are disinfected by washing and disinfecting them with chemicals. Therefore, disinfecting and washing endoscopes is quite a time-consuming procedure. Disinfecting them, then rinsing them with water and rinsing them with disinfecting and/or washing agents, and then drying them takes a lot of time because each step has to be done separately (manually).

The purpose of the invention is to construct a device for automatic washing and disinfection of the application part of endoscopes. Thus, the invention in question solves an existing problem, namely, it reduces the disinfection time and, by automatically controlling the duration of the disinfection process, protects the devices from damage caused by excessive exposure to disinfection fluid.

The invention relates a device for disinfecting and washing flexible and rigid endoscopes, where it is a cuvette, which has the shape of a tube closed from the bottom, is connected to the pump through a hose; at the top the cuvette has a connection to an electronic controller.

Advantageously, the cuvette is attached to the controller.

Advantageously, the device-controller includes a buzzer and a display, advantageously a touch screen.

A method to disinfect and clean flexible and rigid endoscopes in a single disinfection unit as defined above:
- the endoscope is inserted into a cuvette with disinfectant liquid, where the display shows the disinfection data, the duration of the stage is carried out through the user interface,
- after disinfection, with the help of pumps, the used fluid is automatically pumped out by the lower pump into the external container for the used fluid, and the endoscope rinse water is pumped into the cuvette,
- after using disinfectant or rinse water, emptying the external container for the used fluid is done automatically,
- after the disinfecting fluid or rinse water is used up, the external container for the used fluid is refilled automatically.

Advantageously, the endoscope, after the automatic disinfection process in the cuvette, is left in it to dry after the flushing water is released.

Terms used above and in the patent description and claims, have the following meanings:
**Cuvette** - means a container that is a device for disinfecting and washing flexible and rigid endoscopes.
**Disinfection** - a process aimed at destroying all microorganisms (viruses, bacteria, fungi), in order to prevent infection.
**Application part of the endoscope** - the part that is in use during the examination and comes into contact with the patient and biological material.

### Figure description:

**Fig.1** - shows the cuvette from the side.
**Fig.2** - shows the monitor cuvette in front view.
**Fig.3** - shows the monitor cuvette in perspective.
**Fig.4** - shows the monitor cuvette in perspective.
**Fig.5** - shows the box with pumps and hoses (4).
**Fig.6** - shows the cuvette with the endoscope.

The invention is illustrated by the following performance example, which is not a limitation of the invention.

### Example:

The entire disinfection process is carried out in a single device - a cuvette (1) - without pulling out the endoscope.

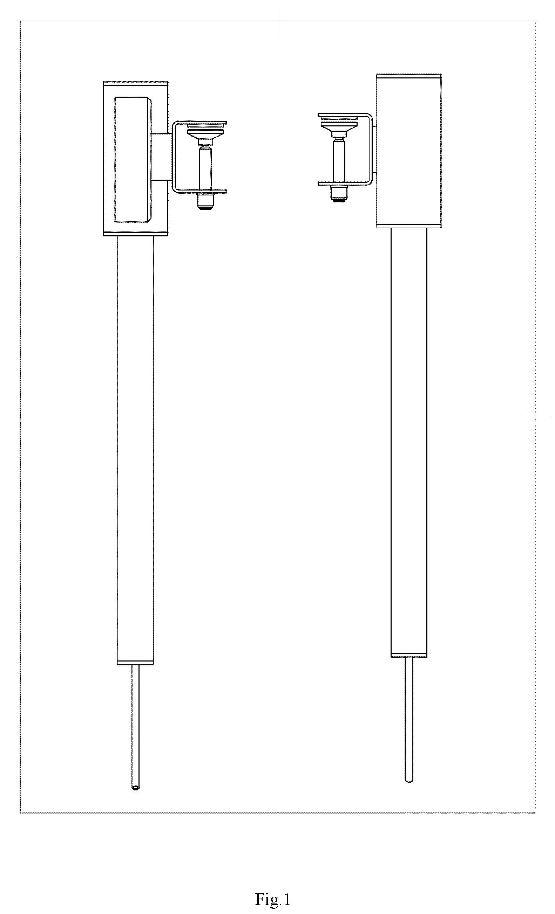

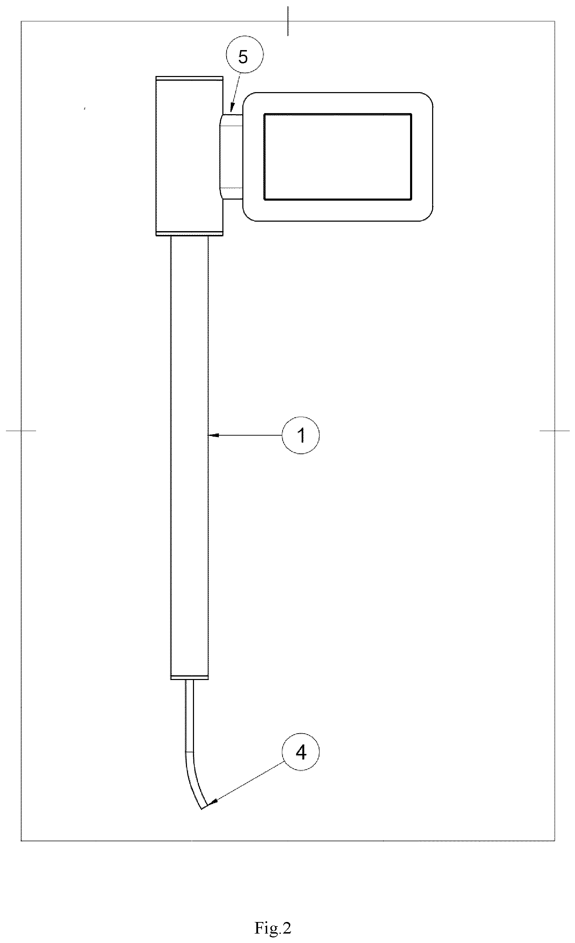

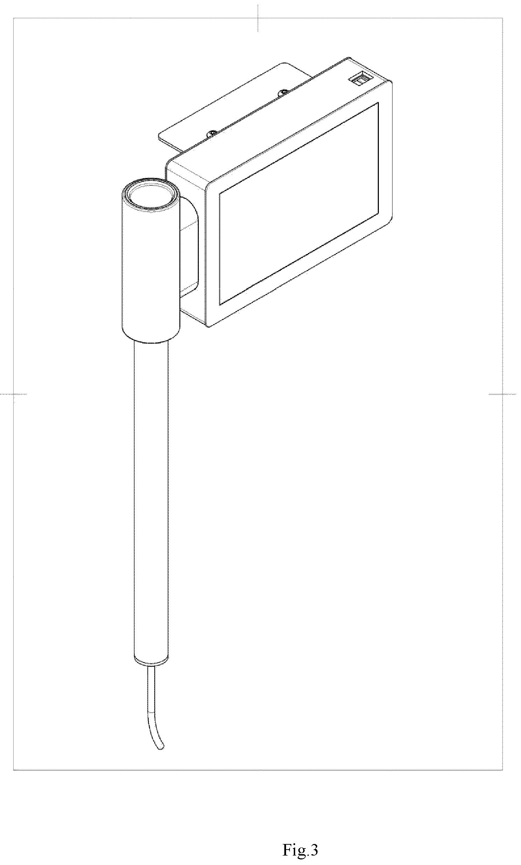

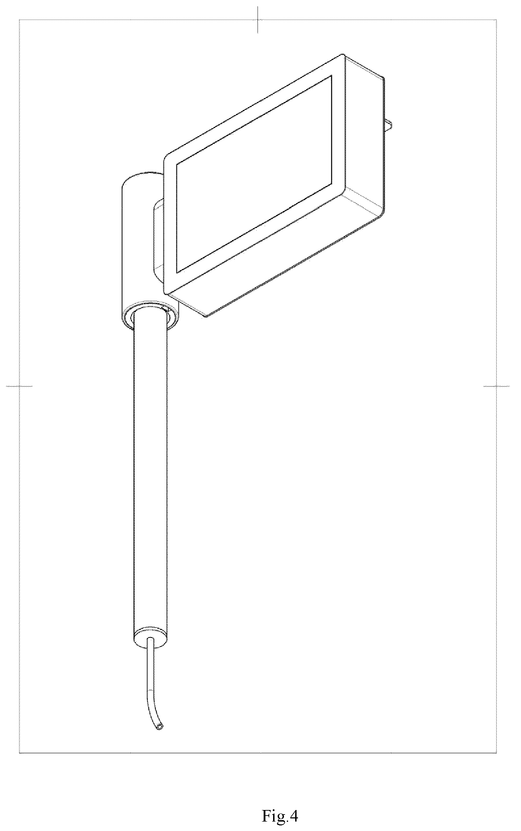

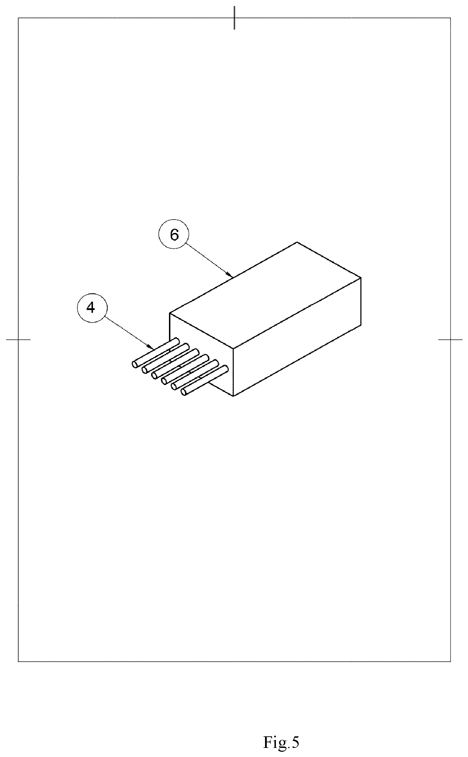

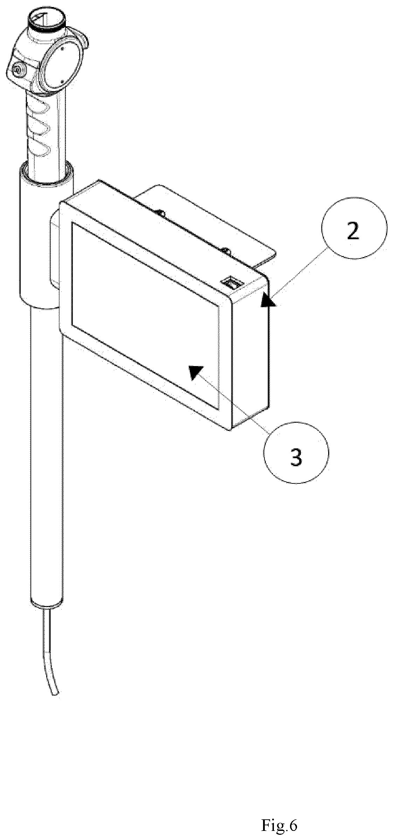

The endoscope is inserted into the cuvette (1) with disinfecting fluid (fig. 1-6). The cuvette (1) is permanently attached to the controller (2). The cuvette (1) is shaped like a tube closed at the bottom, where the used disinfectant fluids and rinse water are drained and discharged through a hose (4). The cuvette (1) at the top by means of a handle (5) is connected to an electronic device (controller with a screen) (3), which indicates (counts down) the time needed for the disinfection process (depending on the liquid used) and the given disinfection stage. The device (3) contains a display that shows all the data.

After disinfection (the endoscope is in the cuvette), with the help of pumps (6), the used fluid is automatically pumped out through the lower pump into the external container for the used fluid, and endoscope rinse water is pumped into the cuvette (1). The water is then discharged, using the pumps (6) into the external container for used water.

The endoscope, after the disinfection process is automatically performed in one devicecell (1), is left in it to dry after the rinse water is released.

The electronic device (3) indicating the parameters, i.e. disinfection stage, the duration of a given stage, is implemented via a user interface.

The device's display (3) is equipped with a touch screen and buzzer. The detection of the presence of the endoscope and the measurement of the fluid level is implemented in the cuvettes (1).

In cuvettes (1), similarly, identification for the presence of the endoscope takes place, i.e. the process starts automatically when the endoscope is put down in cuvette (1).

The controller (2/3) allows selection of the disinfection fluid and automatically adjusts the disinfection process times based on this selection.

## Claims

1. A device for disinfection and cleaning of flexible and rigid endoscopes, **characterized in that** it is a cuvette (1), which has the shape of a tube closed from the bottom, is connected to the pump (6) through a hose (4); at the top, the cuvette (1) has a connection (5) with an electronic controller (3).

2. The device according to claim 1, **characterized in that** the cuvette (1) is attached to the controller (2).

3. The device according to claim 1, **characterized in that** the device (3) contains a buzzer and a display, advantageously a touch screen.

4. A method for disinfecting and washing flexible endoscopes and rigid endoscopes, **characterized in that** in one disinfection device defined in claim 1:
- The endoscope is inserted into a cuvette with disinfectant liquid, where the display shows the disinfection data, the duration of the stage is carried out through the user interface,
- After disinfection, with the help of pumps, the used fluid is automatically pumped out by the lower pump into the external container for the used fluid, and the endoscope rinse water is pumped into the cuvette,
- After using disinfectant or rinse water, emptying the external container for the used fluid is done automatically,
- After the disinfecting fluid or rinse water is used up, the external container for the used fluid is refilled automatically.

5. The method according to claim 4, **characterized in that** the endoscope, after the automatic disinfection process in the cuvette, is left in it to dry after releasing the rinse water.
